# EUROPEAN PATENT SPECIFICATION

(11) **EP 3 538 085 B1**
(45) Date of publication and mention of the grant of the patent: **12.07.2023**
(21) Application number: 17869492.3
(22) Date of filing: 11.10.2017
(51) Int. Cl.: A61K 31/216, A61K 36/534, A23L 2/00, A61P 25/28

(54) **USE OF A SPEARMINT EXTRACT FOR IMPROVING THE RATE OF NEUROGENESIS**
VERWENDUNG EINES SPEARMINT-EXTRAKTS ZUR VERBESSERUNG DER NEUROGENESEGESCHWINDIGKEIT
UTILISATION D'UN EXTRAIT DE MENTHE VERTE POUR AMELIORER LE TAUX DE NEUROGENESE

(30) Priority: 11.11.2016 US 201662420994 P
(43) Date of publication of application: 18.09.2019
(73) Proprietor: Kemin Industries, Inc., Des Moines, Iowa 50317 (US)
(72) Inventor: HERRLINGER, Kelli, Ankeny, Iowa 50023 (US); FONSECA, Brenda, Des Moines, Iowa 50304 (US)
(74) Representative: Greaves Brewster LLP
(86) International application number: PCT/US2017/056165
(87) International publication number: WO 2018/089157

(56) References cited:
- US-A1- 2014 044 813
- US-A1- 2014 196 163
- US-A1- 2014 208 450
- US-A1- 2015 209 317
- US-B2- 7 998 460
- KRISTIN M. NIEMAN ET AL: "Tolerance, bioavailability, and potential cognitive health implications of a distinct aqueous spearmint extract", FUNCTIONAL FOODS IN HEALTH AND DISEASE, vol. 5, no. 5, 30 May 2015 (2015-05-30), pages 165-187, XP055678238, ISSN: 2378-7007, DOI: 10.31989/ffhd.v5i5.181
- B. NARASIMHAMOORTHY ET AL: "Differences in the chemotype of two native spearmint clonal lines selected for rosmarinic acid accumulation in comparison to commercially grown native spearmint", INDUSTRIAL CROPS AND PRODUCTS., vol. 63, 1 January 2015 (2015-01-01), pages 87-91, XP055678241, NL ISSN: 0926-6690, DOI: 10.1016/j.indcrop.2014.10.044
- MARTINA CIRLINI ET AL: "Phenolic and Volatile Composition of a Dry Spearmint (Mentha spicata L.) Extract", MOLECULES, vol. 21, no. 8, 1 January 2016 (2016-01-01), pages 1-15, XP055581636, DOI: 10.3390/molecules21081007
- ITO N ET AL: "Rosmarinic acid from Perillae Herba produces an antidepressant-like effect in mice through cell proliferation in the Hippocampus", MEDICINAL & AROMATIC PLANTS ABSTRACTS, SCIENTIFIC PUBLISHERS, SCIENTIFIC PUBLISHERS, NEW DELHI - INDIA, vol. 30, no. 6, 1 December 2008 (2008-12-01), XP018025714, ISSN: 0250-4367
- PARK SE JIN ET AL: "Standardized Prunella vulgaris var. lilacina Extract Enhances Cognitive Performance in Normal Naive Mice : Memory Enhancing Effect of Prunella vulgaris", PHYSIOTHERAPY RESEARCH, vol. 29, no. 11, 17 September 2015 (2015-09-17), pages 1814-1821, XP055926369, GB ISSN: 0951-418X, DOI: 10.1002/ptr.5449 Retrieved from the Internet: URL:https://api.wiley.com/onlinelibrary/td m/v1/articles/10.1002%2Fptr.5449>

## Description

### FIELD OF THE INVENTION

The present invention is defined in the appended claims. The present disclosure relates generally to botanical extract compositions and methods of using those compositions in order to improve brain health through enhanced neurogenesis. More particularly, the present disclosure relates to using spearmint (*Mentha spicata* L.) extracts in order to increase the rate of neurogenesis.

### BACKGROUND OF THE INVENTION

The area of the hippocampus known as the dentate gyrus is one of the few areas in the adult human brain that can produce new neurons throughout the lifespan. This process of producing new neurons is known as neurogenesis Gage, FH, Science 287: 1433-1438 (2000), Spalding et al., Cell 153: 1219 - 1227 (2013). In contrast to synapotogenesis, the growth of new synapses (the part of the neuron that communicates with another neuron), neurogenesis relates to the growth of new neurons. Researchers have suggested that aging, stress and/or sleep deprivation may decrease the rate of neurogenesis through reduced activity in the dentate gyrus, which results in reduced cognitive performance in healthy adults. Artegiani B, et al. Aging 4: 176-186 (2012), Veena J J, Nat Sci Biol Med 2:26-37 (2011). Evidence suggests that this may happen much earlier in middle age far before the onset of old age. For example, studies in animal models have found that deterioration of the pathways for neurogenesis are already in place by middle age, Hamilton LK, Eur J of Neuro 37:1978-1986. Additionally, Mathews et al., Aging Cell 16: 1195-1199 (2017) found decreased expression of genes involved in the proliferation and early maturation stages of neurogenesis could occur in certain individuals after the age of 20.

Plant-based dietary supplements capable of neurotrophic, antioxidant, neuroprotective and anticholinesterase activity have been investigated in clinical trials for the potential to enhance cognitive function in healthy volunteers. Snitz BE, et al. JAMA. 302:2663-2670 (2009); Reay JL, et al. J Psychopharmacol. 19:357-365 (2005); Kennedy DO, et al. Pharmacol Biochem Behav. 79:401-411 (2004); Pengelly A, et al. J Med Food. 15:10-17 (2012). However, prior research has primarily focused on the effects of administering synthetic compounds, such as synthetic rosmarinic acid. The results of these studies have been mostly inconclusive; moreover, it is not clear that a botanical extract with a large number of active compounds, in addition to active metabolites, would behave in a similar manner. Ito N, et al. Biol Pharm Bull. 31:1367-1380 (2008); Nie H, et al. Prog in Neuro Psychopham & Biol Psych. 51:16-22 (2014); Jin X, et al. Neurochem Res. 38:1828-1837 (2013); Ghaffari H, et al. Life Sci. 113:7-13 (2014); Fonteles AA, et al. Beh Brain Res. 297:91-103 (2016). For instance, several studies only saw effects of neurogenesis when synthetic rosmarinic acid was administered to stressed animals, with no effect in the control, non-stressed animal or culture conditions. *See, e.g.,* Ghaffari H, et al. Life Sci. 113:7-13 (2014); Jin X, et al. Neurochem Res. 38:1828-1837 (2013); Nie H, et al. Prog in Neuro Psychopham & Biol Psych. 51:16-22 (2014).

Spearmint extract is a polyphenol-rich botanical extract containing greater than 60 polyphenolic compounds including rosmarinic acid, salvianolic acid A, salvianolic acid B, lithospermic acid, and caftaric acid, among dozens of others. Cirlini, M., et al. Molecules. 2016; 21:1007. For instance, certain varieties of spearmint extract contain elevated levels of rosmarinic acid (RA), which is an ester of caffeic acid and 3,4-dihydroxyphenylacetic acid. It is also a secondary metabolite of various plant species including those of Lamiaceae. Although rosmarinic acid was first extracted from rosemary (*Rosmarinus officinalis* L.), unlike the other abundant antioxidant compounds of rosemary (carnosic acid and carnosol), rosmarinic acid is more polar and appropriate for use in other food products. There is increased interest in developing products based on the more polar rosmarinic acid that will likely have greater antioxidative efficacy in beverages, sauces, and emulsions. In addition, this molecule is known to have unique properties including antiviral, antibacterial, and anti-inflammatory activities (Mazumder A, Neamati N, Sunder S, Schulz J, Pertz H, Eich E, and Pommier Y. 1997. Curcumin analogs with altered potencies against HIV-1 integrase as probes for biochemical mechanisms of drug action. Journal of Medical Chemistry. 40:3057-3063; Szabo E, Thelen A and Paterson M. 1999. Fungal elicitor preparations and methyl jasmonate enhance rosmarinic acid accumulation in suspension cultures of Coleus Blumei. Plant Cell Reports 18: 485-489; Hooker CW, Lott WB and Harrich D. 2001. Inhibitors of human immunodeficiency virus Type 1 reverse transcriptase target distinct phases of early reverse transcription. Journal Virology. 75: 3095-3104).

Previous studies have shown that administering botanical extracts, such as mint extracts, can improve cognitive performance in healthy adults. Therefore, an objective of the present study was to determine if botanical extracts, such as mint extracts, could enhance neurogenesis or increase the growth rate of new neurons. Further still, an objective of the present study was to evaluate botanical extracts from proprietary *Mentha spicata* L. including KI-MsEM0110 and KI-MsEM0042 that have elevated levels of polyphenols, excellent vigor and overall agronomic robustness.

It is worth noting, spearmint extract has been shown to function differently than rosmarinic acid alone. For instance, although rosmarinic acid is the polyphenol compound of highest concentration in the proprietary *Mentha spicata* L. lines KI-MsEM0110 and KI-MsEM0042, a recent study showed that the spearmint extract performed significantly differently than the equivalent amount of rosmarinic acid in isolation. Fonseca BA, et al. FASEB J. 31:lb367 (2017).

Accordingly, persons of ordinary skill in the art would appreciate that a botanical extract that contains a complex mixture of polyphenols could interact and provide synergies that are not available when a single molecule acts alone. For example, some of the interactions at different dosage amounts could make rosmarinic acid more or less efficacious. Here, the researchers have unexpectedly determined that orally administering spearmint extract leads to improved brain health through a statistically significant increase in the rate of neurogenesis.

### BRIEF SUMMARY OF THE INVENTION

The present invention is defined in the appended claims. The present disclosure relates generally to botanical extract compositions rich in polyphenols and methods of using those compositions in order to improve brain health through enhanced neurogenesis. Another aspect of the present disclosure relates to methods of using spearmint (mentha spicata) extract for enhanced neurogenesis.

Further still, another aspect of the present invention relates to using botanical extracts from Kemin Industries Inc.'s proprietary spearmint (*Mentha spicata* L. including lines named KI-MsEM0110 and KI-MsEM0042, which are described in detail in U.S. Patent Nos. 9,545,075 and 9,545,076) in order to improve brain health through enhanced neurogenesis. For instance, Neumentix^{™} Phenolic Complex K110-42 (Neumentix) is a natural extract derived from spearmint containing greater than 60 phenolic constituents (Cirlini, M., et al. Molecules. 2016; 21:1007) that has been shown to improve working memory after 90 days of supplementation in healthy adults with age associated memory impairment. Fonseca et al. Amer. Acad. of Neur. Conf. Washington D.C. (2015).

### BRIEF DESCRIPTION OF THE FIGURES

FIG. 1 depicts the plasma levels of rosmarinic acid after administering Neumentix.
FIG. 2 depicts the study design and timeline
FIG. 3 depicts the labeled hippocampal neurons
FIG. 4A and 4B depict the effects of Neumentix on neurogenesis or primary rat hippocampal neurons.

### DETAILED DESCRIPTION OF THE INVENTION

The present invention is defined in the appended claims. As disclosed in greater detail herein, the present disclosure relates to botanical extract compositions and methods of using those compositions in order to improve brain health through enhanced neurogenesis. Another aspect of the present disclosure relates to methods of using spearmint (*Mentha spicata* L.) extract for enhanced neurogenesis.

Cognitive performance can decrease due to aging, stress and/or sleep deprivation. Alhola, P, et al. Neuropsych Dis & Treat. 3:553-567 (2007); de Kloet ER, et al. TINS 22:422-424 (1999); Verhaeghen P, Psych Bull. 122:231-249 (1997). One possible explanation for this reduced cognitive performance is that neurogenesis, the process of producing new neurons, decreases under these conditions Artegiani B, et al. Aging 4: 176-186 (2012), Veena J J, Nat Sci Biol Med 2:26-37 (2011). Botanical extracts, such as the spearmint extract, which according to at least one embodiment of the present invention contains greater than 66 phenolic constituents, have been clinically shown to improve cognition including working memory

By way of background, a SAMP8 animal study found that the spearmint extract led to increased performance on a novel object recognition task along with T-maze acquisition and retention (Farr, SA. et al. Physio & Beh. 165:328-338 (2016)). These tasks are considered to be hippocampal-dependent tasks. In addition, the study revealed decreased levels of several oxidative stress markers in the hippocampus on the animals given the spearmint extract. Both of these findings suggest that the spearmint extract could play a potential role in increasing the function/performance of the hippocampus with one potential mechanism of action being an increased rate of neurogenesis.

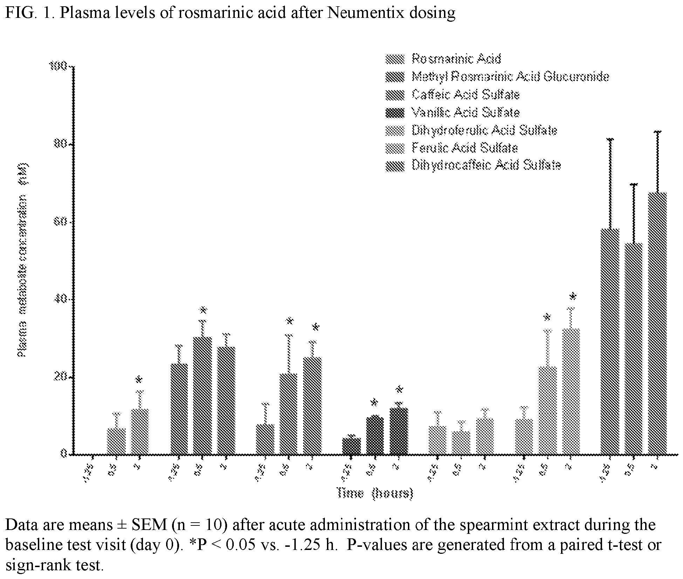

In a follow up, single-arm human clinical trial, the researchers found that administration of a similar (human equivalent) dose allowed subjects to perform better on several cognitive tasks including a polygon matching task that is also known to rely heavily on hippocampal performance. Nieman, K. et al. Funct Foods Health & Disease. 5(5): 165-187 (2015). For instance, the single-arm study measured the acute (~2 to 4 hour post-supplementation) and chronic (30 days post-supplementation) improvements in cognition with daily consumption of 900 mg Neumentix, which coincided with the increased plasma rosmarinic acid levels, as shown in FIG. 1.

In a larger, randomized, double-blind, placebo controlled study the researchers found that individuals taking the spearmint extract for 90 days performed significantly better on a spatial working memory task compared to individuals who consumed a placebo. Fonseca et al. Amer. Acad. of Neur. Conf. Washington D.C. (2015). Again this is a hippocampal dependent learning task. Spellman, T. Nature. 522. 309-314 (2015); Wirt, RA. Brain Sci. 7(43):1-21 (2017).

Finally, in another randomized, double-blind placebo controlled study, young healthy men and women were supplemented daily with the spearmint extract, the individuals taking the spearmint extract scored numerically higher on a simple n-back working memory task compared to individuals taking placebo. (Falcone, P. et al. ISSN (2017).

These results suggest that the improved working memory, as shown in previous clinical trials following administration of Neumentix, may be due to increasing rates of neurogenesis in the hippocampus.

The present disclosure relates to administering spearmint extract and its ability to impact the function and performance of the hippocampus by increasing the rates of neurogenesis, for instance increasing the growth of new neurons in an amount that is statistically significant. More specifically, the researchers have determined that orally administering spearmint extract in a daily dose up to 1200 mg, for instance between 300 to 1200 mg each day could increase the rate of neurogenesis. According to at least one embodiment, the spearmint extract is administered in a dosage between 450 to 1000 mg. In another embodiment, the spearmint extract is administered in a dosage of 600 or 900 mg.

In at least one embodiment as an example, spearmint extract was administered daily over a period of at least 30 days. In another embodiment, the extract was administered daily for at least 90 days. In a further embodiment, the extract was administered chronically for a period that exceeded 90 days.

In at least one embodiment as an example, the oral administration occurs once a day, however the dosing could occur in smaller doses throughout the day. Persons of ordinary skill in the art will appreciate various methods of orally administering the spearmint extract, including but not limited capsules, tablets, soft gels, gummies, drink sticks, shots, ready-to-drink beverages, chocolate bites, coffee single serve "k-cups", tea, effervescent wafers, gummies, and food bars.

Another aspect of present disclosure relates to methods of administering botanical extracts from proprietary *Mentha spicata* L. including KI-MsEM0110 and KI-MsEM0042 that have elevated polyphenol and rosmarinic acid levels, excellent vigor and overall agronomic robustness. Narasimhamoorthy B. Ind Crops & Prod. 63:87-91 (2015). These proprietary lines are described in U.S. Patent Nos. 9,545,075 and 9,545,076, the disclosures of which are expressly incorporated herein in their entirety.

In at least one embodiment, the plant KI-MsEM0110 produces rosmarinic acid comprising greater than 8% dry weight rosmarinic acid and preferably greater than 10% dry weight rosmarinic acid. The *Mentha spicata* L. plant denominated KI-MsEM0110 as produced by a spearmint line deposited under the terms of the Budapest Treaty with the ATCC on November 5, 2015 and assigned accession number PTA-122652.

In another embodiment, the plant KI-MsEM0042 produces rosmarinic acid comprising greater than 8% dry weight rosmarinic acid and preferably greater than 10% dry weight rosmarinic acid. The *Mentha spicata* L. plant denominated KI-MsEM0042 as produced by a spearmint line deposited under the terms of the Budapest Treaty with the ATCC on November 5, 2015 and assigned accession number PTA-122651.

### EXAMPLES

In order to determine whether botanical extracts, such as spearmint extract, could enhance neurogenesis, the investigators conducted a trial studying rat hippocampal cells at physiologically relevant concentrations in a cell culture assay. The study was performed at QPS Austria GmbH, Parkring 12, 8074 Grambach, Austria, Study Number C021340.

Materials: Neumentix (Kemin Foods, L.C., Des Moines, IA), a spearmint extract available in powder form, was dissolved in culture medium to the following final concentrations of rosmarinic acid (RA): 10 nM, 100 nM, 1 µM, and 10 µM.

The spearmint extract was sourced from two proprietary spearmint plants KI-110 and KI-42 through traditional breeding practices (Narasimhamoorthy, B. et al. Indust Crops and Prod 63:87-91 (2015)). The proprietary spearmint crop was grown in the United States in accordance with Good Agricultural Practices (GAP). The harvested leaves were dried and milled prior to extraction. In the extraction step, the dried milled leaves were mixed with water and phosphoric acid (processing aid to assist in the extraction of the phenolic constituents from plant tissue), heated, filtered and dried to obtain the dry spearmint extract. The dry spearmint extract is manufactured in compliance with current Good Manufacturing Practices (cGMP) for food (21 C.F.R. 110) and substantially free of solvents. Lasrado JA et al. Reg Toxicol and Pharma 71:213-224 (2015).

The dry spearmint extract consists of a blend of polyphenols including key molecules of rosmarinic acid and its derivatives along with smaller amounts of salvianolic acids, caffeoylquinic acids, hydroxybenzoic acids, hydroxycinnamic acids, flavones, and flavanones. The phenolic fraction of the spearmint extract has been fully characterized by means of ultra-high performance liquid chromatography-electrospray ionization-mass spectrometry (UHPLC-ESI-MS) revealing a total of 66 compounds in the extract. The total amount of the polyphenol blend in the spearmint extract analyzed on the basis of UHPLC-ESI-MS was 263 mg/g. As described in Table 1, an analysis of the percentages of specific polyphenols with the extract demonstrates that rosmarinic acid makes up about 88% of the total amount of detected polyphenols, followed by salvianolic acids at 6% and caffeoylquinic acids at 1% along with the hydroxycinnamic acids (including caftaric acid) also at about 1%.

**Table 1. Quantitative results (mg/g sample) for polyphenolic fraction of the spearmint extract.**

| Quantified as... | Concentration(mg/g) | Percent (%) |
|---|---|---|
| Rosmarinic acid derivatives | 230.50 ± 13.5 | 87.653% |
| Salvianolic acids | 14.70 ± 1.19 | 5.590% |
| Caffeoylquinic acids | 3.06 ± 0.27 | 1.164% |
| Hydroxycinnamic acids | 3.00 ± 0.36 | 1.141% |
| Hydroxyphenylpropanoic acids | 0.99 ± 0.10 | 0.376% |
| Hydroxybenzoic acids | 0.61 ± 0.08 | 0.232% |
| Flavones | 0.53 ± 0.02 | 0.202% |
| Flavanones | 0.04 ± 0.01 | 0.015% |
| Flavonols | 0.01 ± 0.00 | 0.004% |
| | | |
| | | |
| Total Phenolics | 262.97 ± 15.90 | |

Source: Cirlini, M. et al. Mol 21:1007 (2016).

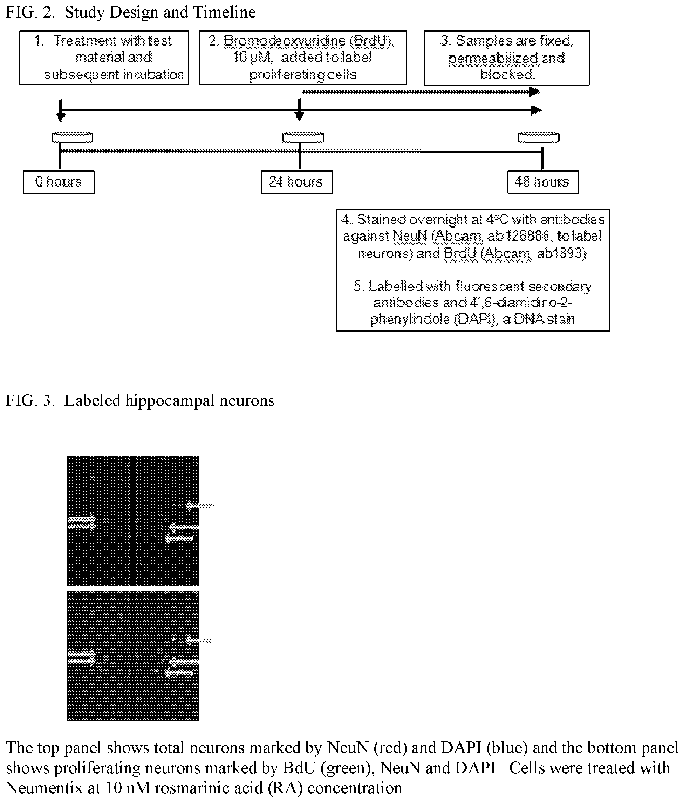

Methods: Primary hippocampal neurons were prepared from time mated Sprague Dawley rats at embryonic day 18. Hippocampi were isolated from embryos, prepared, suspended in culture medium and 1.25 × 10⁴ cells were seeded on individual coverslips and cultured at 37°C and 5% CO2. After adherence, cells were treated with Neumentix, vehicle (culture medium), or fibroblast growth factor (FGF) (positive control) and incubated as described in Figure 2, with abbreviations listed below in Table 2.

**Table 2. Abbreviations.**

| | |
|---|---|
| BrdU | Bromdesoxyuridine |
| DAPI | 4',6-diamidino-2-phenylindole |
| SD | Standard deviation |
| DIV | Days in vitro |
| E18 | Embryonic day 18 |
| FGF | Fibroblast growth factor |
| GLP | Good laboratory praxis |
| HEPES | 4-(2-hydroxyethyl)-1-piperazineethanesulfonic acid |
| HCI | Hydrochloric acid |
| NaHC03 | Sodium bicarbonate |
| PBS | Phosphate buffered saline |
| RA | Rosmarinic acid |
| RT | Room temperature |
| SEM | Standard error of mean |
| SOP | Standard operating procedure |
| Vc | Vehicle control |

Rat primary hippocampal cells were treated in vitro with Neumentix at the following doses (based on rosmarinic acid concentration) 10 nM, 100 nM, 1 uM, or 10 uM vehicle, or Fibroblast Growth Factor. Images were analyzed for the percentage of proliferating neurons (defined by positive staining for Bromdesoxyuridine (BrdU), NeuN and 4',6-diamidino-2-phenylindole (Dapi)) compared to the total number of neurons (defined as NeuN and Dapi positive cells).

The data was analyzed by one-way ANOVA analysis followed by Fisher's LSD post hoc test using GraphPad Prism 4 and 6. FGF was not included in the statistical evaluation. Data are presented as Mean ± SEM.

Mosaic images (6.1 mm × 4.7 mm) around the center of the coverslip were recorded on a Zeiss AxioImager.Z1 microscope. 11-12 coverslips were evaluated per condition.

The number of proliferating neurons (BrdU, NeuN, and DAPI positive) as a percentage of the total number of neurons (NeuN and DAPI positive) was determined by rater-independent macro-based image analysis using the ImagePro Plus (v6.2) software.

Results: An overall statistically significant treatment effect (p=0.017) was identified. Further, a pairwise comparison showed Neumentix at a 10 nM dose group (0.02083 mg/L Neumentix) had significantly greater (p=0.023) levels of neurogenesis than vehicle treated cultures.

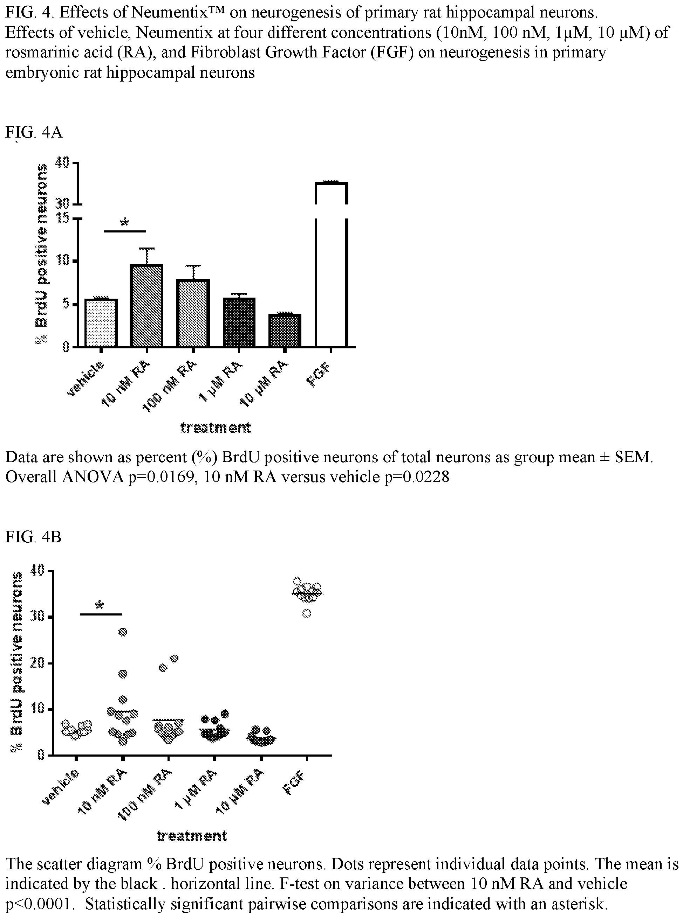

More specifically, primary embryonic hippocampal neurons responded differentially based on the concentration of Neumentix applied to the cells with a statistically significant overall treatment effect, F(4,53)=3.317, p=0.0169 (FIG. 4A).

Pairwise comparisons indicated that the Neumentix at the 10 nM RA condition demonstrated significantly greater amounts of neurogenesis compared to the vehicle control, p=0.0228 (FIG. 4A). Contrary to what would be expected by a person of ordinary skill in the art, higher levels of RA at 100nM to 10µM concentrations did not show an increased benefit (were not statistically significant when compared to the vehicle control with p>0.05).

Results obtained from neurons treated with Neumentix at a concentration of 10 nM or 100 nM RA showed a larger spread of individual data points compared to all other groups which was found to be statistically significant, p<0.0001 (FIG. 4B).

Conclusion: This study demonstrated that Neumentix, a water soluble spearmint extract, administered at levels of rosmarinic acid (10 nM) equivalent to that detected in the plasma of subjects following oral supplementation with 900 mg Neumentix, can significantly increase neurogenesis in primary rat hippocampal cells. Interestingly, the researchers have demonstrated a dose response curve in the opposite direction, in that the rates of neurogenesis decreased as the concentrations increased.

Studies conducted to look at the effects of rosmarinic acid on markers of new neural growth have used concentrations of the isolated molecule that are several orders of magnitude higher in concentration than the concentrations used in the current study. For example, Ghaffari H. Life Sci. 113:7-13 (2014) investigated effects of rosmarinic acid in isolation at concentrations from 5 to 100 µM, while the current study was able to demonstrate increased rates of neurogenesis at concentration in the nM range, or a thousand times less concentrated. In addition, many of the studies have observed increased neurogenesis rates as concentrations increased. Ito N, et al. Biol Pharm Bull. 31:1367-1380 (2008); Nie H, et al. Prog in Neuro Psychopham & Biol Psych. 51:16-22 (2014); Jin X, et al. Neurochem Res. 38:1828-1837 (2013); Ghaffari H, et al. Life Sci. 113:7-13 (2014). Thus, the present invention stands in contrast to those studies, demonstrating that administering a phenolic-rich botanical extract such as spearmint extract can achieve increased rates of neurogenesis at significantly lower concentrations.

## Claims

1. A water-soluble spearmint extract for use in increasing the rate of neurogenesis in a patient in need thereof, wherein the spearmint extract is in the form of a dry powder and is administered at least once a day in an amount ranging from 300 to 1200 mg, wherein said spearmint extract comprises *Mentha spicata* L. line KI-MsEM0110 which has been deposited with ATCC and assigned accession number PTA-122652 and/or *Mentha spicata* L. line KI-MsEM0042 which has been deposited with ATCC and assigned accession number PTA-122651.

2. A spearmint extract for use according to claim 1, wherein the extract is administered orally.

3. A spearmint extract for use according to claim 1 or 2, wherein the spearmint extract is administered at least once a day in an amount ranging from 450 to 900 mg.

4. A spearmint extract for use according to any one of claims 1 to 3, wherein the spearmint extract is essentially free from solvents.

5. A spearmint extract for use according to any one of the preceding claims, wherein the spearmint extract is administered as a capsule, tablet or soft gel.

6. A spearmint extract for use according to any one of the preceding claims, wherein the spearmint extract is added to a food or beverage.

## Patentansprüche

1. Wasserlöslicher Extrakt von grüner Minze zur Verwendung bei der Erhöhung der Neurogenese-Rate in einem Patienten, der dies benötigt, wobei der Extrakt von grüner Minze in Form eines Trockenpulvers vorliegt und mindestens einmal täglich in einer Menge im Bereich von 300 bis 1200 mg verabreicht wird,
wobei der Extrakt von grüner Minze *Mentha spicata* L. Linie KI-MsEM0110, die beim ATCC hinterlegt wurde und die Hinterlegungsnummer PTA-122652 erhalten hat, und/oder *Mentha spicata* L. Linie KI-MsEM0042, die beim ATCC hinterlegt wurde und die Hinterlegungsnummer PTA-122651 erhalten hat, umfasst.

2. Extrakt von grüner Minze zur Verwendung nach Anspruch 1, wobei der Extrakt oral verabreicht wird.

3. Extrakt von grüner Minze zur Verwendung nach Anspruch 1 oder 2, wobei der Extrakt von grüner Minze mindestens einmal täglich in einer Menge im Bereich von 450 bis 900 mg verabreicht wird.

4. Extrakt von grüner Minze zur Verwendung nach einem der Ansprüche 1 bis 3, wobei der Extrakt von grüner Minze im Wesentlichen frei von Lösemitteln ist.

5. Extrakt von grüner Minze zur Verwendung nach einem der vorhergehenden Ansprüche, wobei der Extrakt von grüner Minze als Kapsel, Tablette oder Weichgel verabreicht wird.

6. Extrakt von grüner Minze zur Verwendung nach einem der vorhergehenden Ansprüche, wobei der Extrakt von grüner Minze einem Nahrungsmittel oder Getränk zugesetzt wird.

## Revendications

1. Extrait de menthe verte soluble dans l'eau à utiliser pour augmenter le taux de neurogenèse chez un patient en ayant besoin, l'extrait de menthe verte étant sous la forme d'une poudre sèche et étant administré au moins une fois par jour en une quantité allant de 300 à 1200 mg, ledit extrait de menthe verte comprenant *Mentha spicata* L. lignée KI-MsEM0110 qui a été déposée auprès de l'ATCC et a reçu le numéro d'accès PTA-122652 et/ou *Mentha spicata* L. lignée KI-MsEM0042 qui a été déposée auprès de l'ATCC et a reçu le numéro d'accès PTA-122651.

2. Extrait de menthe verte à utiliser selon la revendication 1, l'extrait étant administré par voie orale.

3. Extrait de menthe verte à utiliser selon la revendication 1 ou 2, l'extrait de menthe verte étant administré au moins une fois par jour en une quantité allant de 450 à 900 mg.

4. Extrait de menthe verte à utiliser selon l'une quelconque des revendications 1 à 3, l'extrait de menthe verte étant essentiellement exempt de solvants.

5. Extrait de menthe verte à utiliser selon l'une quelconque des revendications précédentes, l'extrait de menthe verte étant administré sous la forme d'une capsule, d'un comprimé ou d'un gel mou.

6. Extrait de menthe verte à utiliser selon l'une quelconque des revendications précédentes, l'extrait de menthe verte étant ajouté à un aliment ou à une boisson.
